# EUROPEAN PATENT SPECIFICATION

(11) **EP 4 034 216 B1**
(45) Date of publication and mention of the grant of the patent: **21.01.2026**
(21) Application number: 20780610.0
(22) Date of filing: 22.09.2020
(51) Int. Cl.: A61M 25/10, A61B 5/107, A61B 5/00

(54) **BALLOON CATHETER COMPRISING AN INFLATABLE BALLOON AND A CAPACITIVE SENSOR FOR MEASURING A DIAMETER OF THE BALLOON**
BALLONKATHETER MIT EINEM AUFBLASBAREN BALLON UND EINEM KAPAZITIVEN SENSOR ZUR MESSUNG DES BALLONDURCHMESSERS
CATHÉTER À BALLONNET GONFLABLE AINSI QU'UN CAPTEUR CAPACITIF PERMETTANT DE MESURER UN DIAMÈTRE DU BALLONNET

(30) Priority: 26.09.2019 EP 19199773
(43) Date of publication of application: 03.08.2022
(73) Proprietor: BIOTRONIK AG, 8180 Bülach (CH)
(72) Inventor: FARGAHI, Amir, 8180 Bülach (CH)
(74) Representative: WSL Patentanwälte Partnerschaft mbB
(86) International application number: PCT/EP2020/076390
(87) International publication number: WO 2021/058462

(56) References cited:
- DE-U1- 202019 104 513
- US-A1- 2013 123 694
- US-A1- 2014 012 160
- US-B1- 6 179 858

## Description

The invention relates to a balloon catheter for measuring a diameter of a balloon.

When the balloon of a balloon catheter, during a procedure, is in the desired location in the blood vessel (for example in the region of a stenosis in the case of angioplasty), the balloon of the balloon catheter can generally be inflated under high pressure (up to 40 atm) using a fluid inflation medium, which is introduced into the balloon interior, wherein the balloon expands, in particular in the radial direction (that is, perpendicularly to the axial direction of the balloon catheter), and the diameter of the balloon increases accordingly in the radial direction. As a result of the high pressure that the balloon exerts on the vessel wall, the calcium deposits, for example during angioplasty, are pressed into the vessel wall, and the vessel diameter increases. During such a procedure, it is desirable, of course, that the treating physician is able to monitor the diameter of the balloon that arises during the inflation of the balloon (in the radial direction) in a manner that is as controlled and efficient as possible.

For determining or estimating the instantaneous diameter of a balloon of a balloon catheter, it is known in the prior art, for example, to use the relationship between an inflation pressure that is present at the balloon catheter, or present in the balloon interior, and the diameter of the balloon, this relationship usually being depicted in what is known as a compliance chart of the balloon catheter. The inflation pressure can generally be measured, whereby the corresponding diameter of the balloon can be estimated therefrom.

Moreover, balloon catheters are known in which the balloons comprise, for example, printed sensors or strain gauges on the balloon surface. Additional electrical lines, which run between the balloon and a proximal end of the balloon catheter along the catheter shaft (such as printed conductor tracks), are therefore inevitable.

US 6,179,858 discloses an array of capacitive sensors composed of an electrically conductive exterior pad and an electrically conductive interior pad, which are attached to the balloon and can be formed from aluminum foil.

Furthermore, US 2009/041923 discloses a medical device having a lubricious coating on at least a section of the device. The coating is a network of a hydrophilic compound cross-linked to itself and interlocked with a network of a cross-linked polymerized multifunctional monomer or polymer.

US 2013/0123694 Al discloses a method for measuring a balloon expansion profile in vivo. The method comprises providing a balloon with at least one sensing element as a diagnostic device, where the at least one sensing element is characterized by at least one attribute that is representative of balloon dimension; measuring the at least one attribute to obtain an observed attribute value; and estimating the balloon dimension and the balloon expansion profile based on the observed attribute value. A diagnostic kit for measuring a balloon expansion profile in vivo is also provided. The diagnostic kit comprises the diagnostic device; a measurement module for measuring an observed attribute value for the attribute; and a processor module for processing the observed attribute value to estimate the balloon expansion profile as one or more outputs.

US 2014/0012160 A1 discloses an apparatus for medical diagnosis and/or treatment. The apparatus includes a flexible substrate forming an inflatable body and a plurality of force sensing elements disposed on the flexible substrate. The plurality of force sensing elements are disposed about the inflatable body such that the force sensing elements are disposed at areas of minimal curvature of the inflatable body in a deflated state.

DE 20 2019104 513 U1 discloses balloon for a balloon catheter, comprising a wall of the balloon surrounding an inner space of the balloon, wherein the inner space can be supplied with a fluid medium in order to deploy the balloon, characterized in that on the wall of the balloon at least one strain gauge for measuring a diameter of the balloon and/or in that at least one piezoelectric element for influencing a diameter of the balloon is arranged on the wall of the balloon.

Proceeding from this, it is the object of the present invention to provide a balloon catheter that allows the diameter of the balloon of the balloon catheter to be determined in a simple manner, for example during angioplasty.

This object is achieved by a balloon catheter having the features of claim 1. Advantageous embodiments of the balloon catheter are provided in the corresponding dependent claims and will be described hereafter.

According to claim 1, a balloon catheter is disclosed, comprising a balloon having an expandable balloon wall, which surrounds an interior of the balloon, wherein the balloon interior can be filled with a fluid inflation medium for inflating the balloon, so that a thickness of the balloon wall decreases as the diameter of the balloon grows during the inflation of the balloon, due to an expansion of the balloon wall.

According to the invention, it is provided that the balloon catheter comprises a capacitive sensor for determining the diameter, wherein the sensor uses a hydrophilic coating arranged on an outer side of the balloon wall, which becomes electrically conductive upon contact with blood of the patient (for example during angioplasty), and wherein the sensor furthermore uses the balloon wall, which is formed by a dielectric, so that a capacitor having a capacitance is formed when the balloon interior is filled with an electrically conductive inflation medium.

The capacitor of the capacitive sensor thus comprises the hydrophilic coating as well as the volume of the inflation medium present in the balloon interior, which serve as electrodes, wherein the two electrodes are separated from one another by the balloon wall configured as a dielectric.

The capacitance of the capacitor, or capacitive sensor, thus formed changes when the balloon is inflated by way of the inflation medium as a result of a changed diameter of the balloon due to the inflation, since the thickness of the balloon wall decreases as the balloon diameter grows due to the increasing expansion of the balloon wall, and the distance between the two electrodes decreases accordingly. Furthermore, the capacitance also changes when the balloon is deflated as a result of the inflation medium being withdrawn from the balloon interior, since the thickness of the balloon wall increases as the balloon diameter decreases. In this way, the instantaneous diameter of the balloon can be measured during inflation or deflation by determining the instantaneous capacitance of the capacitor, in particular when a change in thickness of the balloon wall takes place.

Within the scope of the present invention, components or spots situated closer to the user (physician) of the balloon catheter along the balloon catheter are referred to as proximal. Accordingly, components or spots situated further away from the user of the balloon catheter are referred to as distal.

According to one embodiment of the invention, it is provided that the balloon extends in an axial direction, wherein the diameter of the balloon runs in a radial direction of the balloon, which runs perpendicularly to the axial direction.

The hydrophilic coating consists of one of the following materials or comprises one of the following materials: hygroscopic materials, such as polyvinylpyrrolidone (PVP), glycosaminoglycans such as hyaluronic acid (HA), poly(ethylene glycol) (PEG).

The electrically conductive inflation medium may be one or more of the following substances: saline solution, in particular 0.9% isotonic saline solution, iodine-based contrast media, such as Visipaue ^{®}.

The balloon wall or the corresponding dielectric may be formed from one or more of the following substances, or comprise these: polyamide, in particular PA12, polyether block amides, in particular PEBAX^{®}, polyethylene terephthalate (PET), polyurethane (PUR, TPU), polyether ether ketone (PEEK), linear polyethylenes, or also combinations thereof in the case of multi-layer balloons.

According to one embodiment of the balloon catheter, it is provided that the sensor comprises an evaluation unit, which is connected to the hydrophilic coating in an electrically conducting manner, and which can be connected, or is connected, to the electrically conductive inflation medium in an electrically conducting manner when the balloon interior is filled with the inflation medium, wherein the evaluation unit is designed to measure the capacitance and to use it for determining the instantaneous diameter of the balloon.

According to one embodiment of the balloon catheter, it is furthermore provided that the balloon catheter comprises a catheter shaft extending in the axial direction, wherein the balloon is connected to a distal end of the catheter shaft, and wherein the catheter shaft surrounds a lumen which is connected to the balloon interior and via which the inflation medium can be introduced into the balloon interior.

According to one embodiment of the balloon catheter, it is furthermore provided that the hydrophilic coating is also arranged on an outer side of the catheter shaft. The coating preferably extends continuously from the balloon onto the catheter shaft.

According to one embodiment of the balloon catheter, it is furthermore provided that a section of the coating on the outer side of the catheter shaft forms a contact point for electrically contacting the hydrophilic coating.

According to another embodiment of the balloon catheter, it is provided that the balloon catheter comprises a contact point for electrically contacting the inflation medium at a proximal end section of the catheter shaft. This contact point may be provided at a Luer connector of the catheter shaft, for example.

According to one embodiment of the balloon catheter, it is furthermore provided that the evaluation unit is connected to the particular contact point in an electrically conducting manner via an electrical conductor.

The coating is advantageously arranged in at least one strip on the balloon. In particular the strip is, or the strips are, oriented substantially along the balloon axis, or wound about the balloon axis, in the longest extension thereof. This design of the invention offers in particular advantages during production, and can be combined with any of the above-described designs. According to the basic idea of this design, the balloon is provided with the hydrophilic coating when in the folded state thereof. So as to keep the insertion profile of a balloon catheter as small as possible, the balloon is folded once or several times (usually 3), wherein the folding is carried out along the balloon axis or wound about the same. The hydrophilic coating is applied onto the balloon after folding in this design. In this way, only the radially outer sides of the fold(s) are automatically provided with the hydrophilic coating, while the radially inner sides of the fold(s) remain uncoated. The balloon deploys during inflation. Depending on the folding, accordingly one or more coated strips result in the process, which are oriented along the longitudinal axis or are wound about the same. The strip or strips may be connected via a shared base (for example, the coating of the catheter shaft), resulting in a single capacitance, which changes with the inflation of the balloon in accordance with the inventive idea. As an alternative, the sum of the changes of the individual capacitances of each strip can be detected as a measure of the inflation of the balloon.

Within the scope of the present application, the balloon axis shall be understood to mean the axis of the cylindrical working area of the balloon. The balloon axis accordingly coincides with the catheter axis. Within the scope of the present application, "oriented substantially along the balloon axis" shall accordingly be understood to mean coatings, having the longest extension thereof form an angle of no more than 10° with the balloon axis. Within the scope of the present application, "wound about the balloon axis" shall be understood to mean a coating, having the longest extension thereof spirally or helically aligned or oriented about the balloon axis/catheter axis. The winding does not have to be completely closed in the process.

The above-described balloon catheter is, in particular, a balloon catheter that is configured to carry out angioplasty in a blood vessel, or to implant a vascular implant, and in particular a stent.

Further, a method for carrying out angioplasty, or for implanting a vascular implant, and in particular a stent, using the described balloon catheter or the associated method, is disclosed.

Embodiments of the invention and further features and advantages of the invention will be described hereafter based on the figure. In the drawing:

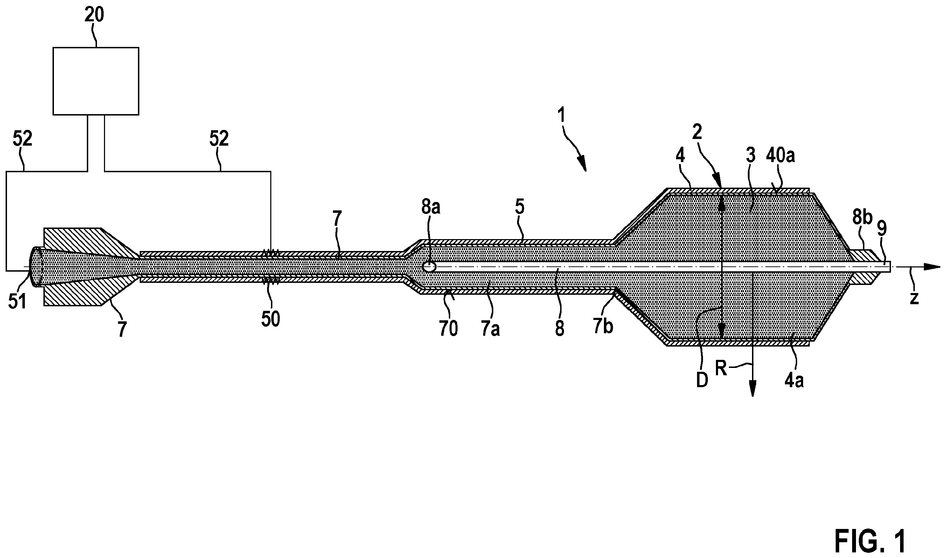

- FIG. 1: shows a schematic illustration of one embodiment of the balloon catheter according to the invention.

As is apparent based on the embodiment according to FIG. 1, for example, the invention relates to a balloon catheter 1, comprising a balloon 4 that extends in a preferably axial direction z and surrounds a balloon interior 4a, which can be filled with a fluid and electrically conductive inflation medium 3 so as to expand the balloon 4, or so as to inflate the balloon 4, in a preferably radial direction R of the balloon 4. The respective radial direction R is situated perpendicularly on the axial direction z, and points outwardly toward the balloon wall 40 of the balloon 4. The balloon catheter 1 furthermore comprises a catheter shaft 7 preferably extending in the axial direction z, wherein the balloon 4 is connected to a distal end 4 of the catheter shaft 7, and wherein the catheter shaft 7 surrounds a lumen 7a which is connected to the balloon interior 4a and via which the inflation medium 3 can be introduced into the balloon interior 4a. The balloon catheter 1 furthermore comprises a capacitive sensor 2 for ascertaining an instantaneous diameter D of the balloon 4, wherein the diameter D extends in particular in the radial direction R. The balloon 4 is preferably cylindrical-symmetrical with respect to the axial direction z.

According to the invention, it is provided that the capacitive sensor 2 comprises a hydrophilic coating 5, which is applied onto the outer side 40a of the balloon wall 40 and which is electrically conductive when wetted with a patient's blood. This blood wetting takes place when the balloon 4 (for example during angioplasty) is inserted into a vessel of the patient.

The sensor 2 furthermore comprises at least one section of the balloon wall 40 that is formed from a dielectric, so that a capacitor having a capacitance is formed when the balloon interior 4a is filled with the electrically conductive inflation medium 3. The coating 5 and the inflation medium 3 then effectively form the electrodes of the capacitor, which as a result of the variable thickness of the balloon wall 40 during inflation or deflation of the balloon 4 has an accordingly variable capacitance (the thickness runs in the radial direction R in FIG. 1). This allows the diameter D of the balloon 4 to be determined, since the expansion behavior of the balloon wall 40, or the functional relationship between the thickness of the balloon wall 40 and the diameter D of the balloon 4, is known. The sensor 2 can thus be calibrated in such a way that the capacitance can serve as a measure of the diameter D.

The sensor 2 furthermore preferably comprises an evaluation unit 20, which is connected to the hydrophilic coating 5 in an electrically conducting manner, and which is connected to the electrically conductive inflation medium 3 in an electrically conducting manner when the balloon interior 4a is filled with the inflation medium 3, wherein the evaluation unit 20 is designed to measure the capacitance and, for example, use it for determining the instantaneous diameter D of the balloon 4 based on a suitable calibration..

For contacting the hydrophilic coating 5 and the inflation medium 3, the balloon catheter 1 includes a respective contact point 50 and 51 at the outer side 70 of the catheter shaft 7 and at a proximal end section 7c of the catheter shaft 7 (which is formed by a Luer connector, for example), the contact points being connected to the evaluation unit 20 via corresponding electrical lines 52.

The balloon catheter 1 can furthermore have an inner lumen 8 for receiving a guide wire, wherein the inner lumen 8 can extend in the lumen 7a, proceeding from a lateral inlet 8a provided at the catheter shaft 7, through the balloon interior 4a to an outlet 8b, which can be provided at a distal tip 9 of the balloon catheter 1.

Due to the invention, advantageously no additional electrical lines are necessary, which have to extend along the catheter shaft 7 to the balloon 4 in order to electrically contact the sensor 2. Due to the hydrophilic coating 5, which is separated from the inflation medium 3 by the balloon wall 40, a robust capacitive sensor 2 is created. In this way, susceptible electronic structures on the balloon surfaces which are designed as sensors can thus be avoided.

## Claims

1. A balloon catheter (1), comprising:
- a balloon (4) having an expandable balloon wall (40), which surrounds a balloon interior (4a) of the balloon (4), the balloon interior (4a) being fillable with a fluid inflation medium (3) for inflating the balloon (4), so that a thickness of the balloon wall (40) decreases as the diameter (D) of the balloon (4) grows during the inflation of the balloon (4), due to an expansion of the balloon wall (40),
**characterized in that**
the balloon catheter (1) comprises a capacitive sensor (2) for determining the diameter (D), the sensor (2) comprising a hydrophilic coating (5) arranged on an outer side (40a) of the balloon wall (40), which becomes electrically conductive when wetted with blood of a patient, and the sensor (2) furthermore comprising the balloon wall (40), which is formed from a dielectric, so that a capacitor having a capacitance is formed when the balloon interior (4a) is filled with an electrically conductive inflation medium (3).

2. The balloon catheter according to claim 1, **characterized in that** the sensor (2) comprises an evaluation unit (20), which is connected to the hydrophilic coating (5) in an electrically conducting manner, and which is connected to the electrically conductive inflation medium (3) in an electrically conducting manner when the balloon interior (4a) is filled with the inflation medium (3), the evaluation unit (20) being designed to measure the capacitance and use it for determining the diameter (D) of the balloon (4).

3. The balloon catheter according to any one of the preceding claims, **characterized in that** the hydrophilic coating (5) consists of one of the following substances, or comprises one of the following substances: polyvinylpyrrolidone (PVP), hyaluronic acid (HA), polyethylene glycol (PEG).

4. The balloon catheter according to any one of the preceding claims, **characterized in that** the balloon catheter (1) comprises a catheter shaft (7), the balloon (4) being connected to a distal end (7b) of the catheter shaft (7), and the catheter shaft (7) surrounding a lumen (7a) which is connected to the balloon interior (4a) and via which the inflation medium (3) can be introduced into the balloon interior (4a).

5. The balloon catheter according to claim 4, **characterized in that** the hydrophilic coating (5) is also arranged on an outer side (70) of the catheter shaft (7).

6. The balloon catheter according to claim 5, **characterized in that** a section (50) of the hydrophilic coating (5) on the outer side (70) of the catheter shaft (7) forms a contact point (50) for electrically contacting the hydrophilic coating (5).

7. The balloon catheter according to any one of claims 4 to 6, **characterized in that** a contact point (51) for electrically contacting the inflation medium (3) is provided at a proximal end section (7c) of the catheter shaft (7).

8. The balloon catheter according to claims 6 and 7, **characterized in that** the evaluation unit (20) is connected to the particular contact point (50, 51) in an electrically conducting manner via an electrical conductor (52).

9. The balloon catheter according to any one of the preceding claims, **characterized in that** the coating is arranged on the balloon in at least one strip.

10. The balloon catheter according to claim 9, **characterized in that** the strip is, or the strips are, oriented substantially along the balloon axis, or wound about the balloon axis, in the longest extension thereof.

## Patentansprüche

1. Ein Ballonkatheter (1), aufweisend:
- einen Ballon (4) mit einer expandierbaren Ballonwand (40), die einen Balloninnenraum (4a) des Ballons (4) umgibt, wobei der Balloninnenraum (4a) mit einem fluiden Inflationsmedium (3) zum Inflatieren des Ballons (4) befüllbar ist, so dass die Dicke der Ballonwand (40) mit zunehmendem Durchmesser (D) des Ballons (4) während des Inflatierens des Ballons (4) aufgrund einer Ausdehnung der Ballonwand (40) abnimmt,
**dadurch gekennzeichnet, dass**
der Ballonkatheter (1) einen kapazitiven Sensor (2) zum Bestimmen des Durchmessers (D) aufweist, wobei der Sensor (2) eine hydrophile Beschichtung (5) aufweist, die auf einer Außenseite (40a) der Ballonwand (40) angeordnet ist, die bei Benetzung mit Blut eines Patienten elektrisch leitfähig wird, und wobei der Sensor (2) ferner die Ballonwand (40) aufweist, die aus einem Dielektrikum gebildet ist, so dass ein Kondensator mit einer Kapazität gebildet wird, wenn der Balloninnenraum (4a) mit einem elektrisch leitfähigen Inflationsmedium (3) gefüllt ist.

2. Der Ballonkatheter nach Anspruch 1, **dadurch gekennzeichnet, dass** der Sensor (2) eine Auswerteeinheit (20) aufweist, die elektrisch leitend mit der hydrophilen Beschichtung (5) verbunden ist und die elektrisch leitend mit dem elektrisch leitfähigen Inflationsmedium (3) verbunden ist, wenn der Balloninnenraum (4a) mit dem Inflationsmedium (3) gefüllt ist, wobei die Auswerteeinheit (20) dazu ausgelegt ist, die Kapazität zu messen und diese zur Bestimmung des Durchmessers (D) des Ballons (4) zu verwenden.

3. Der Ballonkatheter nach einem der vorstehenden Ansprüche, **dadurch gekennzeichnet, dass** die hydrophile Beschichtung (5) aus einer der folgenden Substanzen besteht oder eine der folgenden Substanzen aufweist: Polyvinylpyrrolidon (PVP), Hyaluronsäure (HA), Polyethylenglykol (PEG).

4. Der Ballonkatheter nach einem der vorstehenden Ansprüche, **dadurch gekennzeichnet, dass** der Ballonkatheter (1) einen Katheterschaft (7) aufweist,
wobei der Ballon (4) mit einem distalen Ende (7b) des Katheterschafts (7) verbunden ist und der Katheterschaft (7) ein Lumen (7a) umgibt, das mit dem Balloninnenraum (4a) verbunden ist und über das das Inflationsmedium (3) in den Balloninnenraum (4a) eingebracht werden kann.

5. Der Ballonkatheter nach Anspruch 4, **dadurch gekennzeichnet, dass** die hydrophile Beschichtung (5) auch auf einer Außenseite (70) des Katheterschafts (7) angeordnet ist.

6. Der Ballonkatheter nach Anspruch 5, **dadurch gekennzeichnet, dass** ein Abschnitt (50) der hydrophilen Beschichtung (5) auf der Außenseite (70) des Katheterschafts (7) einen Kontaktpunkt (50) zum elektrischen Kontaktieren der hydrophilen Beschichtung (5) bildet.

7. Der Ballonkatheter nach einem der Ansprüche 4 bis 6, **dadurch gekennzeichnet, dass** ein Kontaktpunkt (51) zum elektrischen Kontaktieren des Inflationsmediums (3) an einem proximalen Endabschnitt (7c) des Katheterschafts (7) vorgesehen ist.

8. Der Ballonkatheter nach den Ansprüchen 6 und 7, **dadurch gekennzeichnet, dass** die Auswerteeinheit (20) über einen elektrischen Leiter (52) elektrisch leitend mit dem jeweiligen Kontaktpunkt (50, 51) verbunden ist.

9. Der Ballonkatheter nach einem der vorstehenden Ansprüche, **dadurch gekennzeichnet, dass** die Beschichtung in mindestens einem Streifen auf dem Ballon angeordnet ist.

10. Der Ballonkatheter nach Anspruch 9, **dadurch gekennzeichnet, dass** der Streifen oder die Streifen im Wesentlichen entlang der Ballonachse ausgerichtet oder um die Ballonachse gewickelt sind, und zwar in ihrer/seiner längsten Ausdehnung.

## Revendications

1. Cathéter à ballonnet (1) comprenant :
- un ballonnet (4) ayant une paroi de ballonnet (40) expansible qui entoure un intérieur de ballonnet (4a) du ballonnet (4), l'intérieur de ballonnet (4a) pouvant être rempli d'un moyen de gonflement fluide (3) pour gonfler le ballonnet (4), de façon qu'une épaisseur de la paroi de ballonnet (40) décroisse à mesure que le diamètre (D) du ballonnet (4) augmente pendant le gonflement du ballonnet (4) à la suite d'une expansion de la paroi de ballonnet (40),
**caractérisé en ce que**
le cathéter à ballonnet (1) comprend un capteur capacitif (2) pour déterminer le diamètre (D), le capteur (2) comprenant un revêtement hydrophile (5) agencé sur un côté extérieur (40a) de la paroi de ballonnet (40), qui devient électriquement conducteur lorsqu'il est humecté par le sang d'un patient, et le capteur (2) comprenant en outre la paroi de ballonnet (40) qui est formée par un diélectrique de façon qu'un condensateur ayant une capacité soit formé lorsque l'intérieur de ballonnet (4a) est rempli d'un moyen de gonflement (3) électriquement conducteur.

2. Cathéter à ballonnet selon la revendication 1, **caractérisé en ce que** le capteur (2) comprend une unité d'évaluation (20) qui est reliée au revêtement hydrophile (5) de façon électriquement conductrice et qui est reliée au moyen de gonflement (3) électriquement conducteur de façon électriquement conductrice lorsque l'intérieur de ballonnet (4a) est rempli du moyen de gonflement (3), l'unité dévaluation (20) étant conçue pour mesurer la capacité et pour l'utiliser pour déterminer le diamètre (D) du ballonnet (4).

3. Cathéter à ballonnet selon l'une des revendications précédentes, **caractérisé en ce que** le revêtement hydrophile (5) est constitué d'une des substances suivantes ou comprend une des substances suivantes : polyvinylpyrrolidone (PVP), acide hyaluronique (HA), polyéthylène glycol (PEG).

4. Cathéter à ballonnet selon l'une des revendications précédentes, **caractérisé en ce que** le cathéter à ballonnet (1) comprend une tige de cathéter (7), le ballonnet (4) étant relié à une extrémité distale (7b) de la tige de cathéter (7) et la tige de cathéter (7) entourant un lumen (7a) qui est relié à l'intérieur de ballonnet (4a) et par lequel le moyen de gonflement (3) peut être introduit dans l'intérieur de ballonnet (4a).

5. Cathéter à ballonnet selon la revendication 4, **caractérisé en ce que** le revêtement hydrophile (5) est également agencé sur un côté extérieur (70) de la tige de cathéter (7).

6. Cathéter à ballonnet selon la revendication 5, **caractérisé en ce qu'**une section (50) du revêtement hydrophile (5) sur le côté extérieur (70) de la tige de cathéter (7) constitue un point de contact (50) pour contacter électriquement le revêtement hydrophile (5).

7. Cathéter à ballonnet selon l'une des revendications 4 à 6, **caractérisé en ce qu'**un point de contact (51) pour contacter électriquement le moyen de gonflement (3) est disposé à une section d'extrémité proximale (7c) de la tige de cathéter (7).

8. Cathéter à ballonnet selon les revendications 6 et 7, **caractérisé en ce que** l'unité d'évaluation (20) est connectée au point de contact particulier (50, 51) de façon électriquement conductrice moyennant un conducteur électrique (52).

9. Cathéter à ballonnet selon l'une des revendications précédentes, **caractérisé en ce que** le revêtement est agencé sur le ballonnet en au moins une bande.

10. Cathéter à ballonnet selon la revendication 9, **caractérisé en ce que** la bande est, ou les bandes sont, orientée(s) sensiblement le long de l'axe du ballonnet ou enroulée(s) autour de l'axe de ballonnet selon l'étendue la plus grande.
